# EUROPEAN PATENT SPECIFICATION

(11) **EP 1 308 177 B1**
(45) Date of publication and mention of the grant of the patent: **11.05.2005**
(21) Application number: 01960753.0
(22) Date of filing: 10.08.2001
(51) Int. Cl.: A61L 27/28, A61M 31/00, A61M 35/00, A61P 19/00

(54) **METHOD FOR THE PRODUCTION OF CHITOSAN-BASED FILMS WITH ENHANCED CELL ADHERING CAPACITY, RESULTING PRODUCT AND APPLICATIONS**
VERFAHREN ZUR HERSTELLUNG VON AUF CHITOSAN BASIERENDEN FILMEN MIT VERBESSERTEM ZELLANHAFTVERMÖGEN, DARAUS RESULTIERENDES PRODUKT AND ANWENDUNGEN
PROCEDE DE PRODUCTION DE FILMS A BASE DE QUITOSANE A CAPACITE D'ADHERENCE CELLULAIRE AUGMENTEE, PRODUIT AINSI OBTENU ET APPLICATIONS

(30) Priority: 10.08.2000 ES 200002057
(43) Date of publication of application: 07.05.2003
(73) Proprietor: Osfarma, S.L., 25007 Lleida (ES)
(72) Inventor: LOPEZ LACOMBA, José Luis, E-28040 Madrid (ES); GARCIA CANTALEJO, Jesus Manuel, E-28040 Madrid (ES); SANZ CASADO, José Vicente, E-28040 Madrid (ES); RAMOS, Viviana Monica, E-28040 Madrid (ES)
(74) Representative: Maldonado Jordan, Julia
(86) International application number: PCT/ES2001/000322
(87) International publication number: WO 2002/011782

(56) References cited:
- EP-A- 0 754 466
- EP-A1- 0 555 807
- EP-A1- 0 784 985
- WO-A1-95/30403
- WO-A1-96/02258
- WO-A1-96/02259
- US-A- 4 326 532
- US-A- 5 116 824
- MUZZARELLI R.A.A. ET AL.: 'Osteoconductive properties of methylpyrrolidinone chitosan in an animal model' BIOMATERIALS vol. 14, no. 12, 1993, pages 925 - 929, XP000616663

## Description

### FIELD OF THE INVENTION

The invention fits into the technical field of production of chitosan based films and its applications in the pharmaceutical, food and biotechnology industries. More specifically, the invention proposes a new method for treatment of chitosan that allows chitosan films to be obtained with increased cell adherence, conferring on it important applications in medicine, pharmacy and biotechnology not achieved until present.

### BACKGROUND OF THE INVENTION

Chitosan is a polymer of natural origin obtained by partial deacetyllation of chitin, homopolymer of β-1, 4-2-acetamide-D-glucosamine, the latter of these being the most abundant polysaccharide in nature after cellulose.

Both chitin and chitosan have important and real applications in the food, pharmaceutical and biotechnology industries. This can be readily seen by reviewing the patents filed on its possible uses in recent years and observing the fundamentally applied nature of International Symposium on the subject (Domard et al., Advances in Chitin Sciences, Vol II, 7^{th} ICCC (Euchis97), Jacques André Publisher (1998); Peter et al., Advances in Chitin Sciences, Vol IV (Euchis 99), Universitat Potsdam (2000)).

Chitosan shows several properties that make its use particularly suitable for the medical/pharmaceutical industry. On the one hand, it is a biocompatible and biodegradable polymer (Lim et al., J. Biomed. Mater. Res. (Appl. Biomater.) 43, 282-290 (1998); Muzzarelli et al., Biomaterials, 14(1), 39-43 (1993)), with antifungal and antimicrobial properties (Sano et al., J. Dental Res., 66, 141-149 (1987); Ramos, V. Doctoral Thesis, Univ. Nacional del Sur, Bahia Blanca, Argentina, (1999)), which may have a large variety of physical states, for example, porous matrices, gels, hydrogels, threads, films, etc., depending on the process to which it is submitted.

On the other hand, it is a product that can immobilise a large number of substances by adsorption and it offers the possibility, given the presence of a large number of reactive groups, of covalently immobilizing, through relatively simple reactions, substances that provide a desired biological activity (bioactive substances), as well as that of their derivatization with different functional groups (WO 92/09635; Muzzarelli and Zattini, J. Biol. Macromol. 8, 137-143 (1986); and Muzzarelli et al. Carbohydr. Polymers 11, 307-320 (1989)).

The references provided indicate the enormous range of possibilities that its use offers in the field of bioengineering, with some important applications already developed such as, for example, its use in the manufacture of medical sutures or as a component of dressing for wounds (US 6,022,556), where some of the aforementioned properties are taken advantage of.

*In vivo* degradation of chitosan produces oligomers of D-glucosamine, whose subsequent degradation allows products to be obtained that can enter the biosynthetic pathway of hyaluronic acid, which makes this product a suitable choice for the guided repair of osseous and osseouscartilaginous injuries. Its positive effect for treatment of this type of injury is disclosed in the literature.

However, up to date, there is no chitosan based product or derivates thereof available in the market within the field of guided tissular repair, especially osseous and osseouscartilaginous lesions.

The reason can be found in a lack of suitability of chitosan for homogeneous cell adhesion and proliferation on a three-dimensional matrix of this compound, one of the features that the matrices used in tissue engineering should possess (Atala and Mooney, Synthetic Biodegradable Polymer Scaffolds, Birkäuser, Boston (1997)). In fact, if a research is performed of the biomedical applications of chitosan, its fundamental use, outside those already mentioned of a suture material and a component of dressing for injuries, lies in its use as a filling material, perhaps acting as a binding agent in the form of hydrogel, or as an encapsulated bioactive substances releasing agent.

Only one Patent was found in North American Patent database corresponding to the last 20 years, in which chitosan is used as a support for guided regeneration and, in this Patent, the early mentioned drawback is overcome by coating the three-dimensional gauze of chitosan with a polylactic-coglycolic film (US 5,830,493), losing the capability for fixation and adsorption of bioactive substances of interest, mentioned earlier, during the process.

On the other hand, if, the filmforming features are added to this activation capability by means of the adsorption or binding of compounds of biological interest, a material would be obtained, in principle, that would be suitable for coating prosthesis, implants and even three-dimensional gauzes of other polymers that can be used in tissue engineering, so that the desired biological action induced by this activated chitosan would be produced precisely in the selected region of the organism.

Once again, a literature search shows an absence in terms of exploitation of this filmforming capacity in this field; the only thing to note is its use as a coating material for dialysis systems (US 5,885,609), treating this material so that its possible cell adhesion is reduced.

There are two main drawbacks for the use of chitosan in the indicated sense. On the one hand, the stabilization process normally used for chitosan films may negatively affect the structure of the support to be coated. In this sense, it should be bared in mind that chitosan normally becomes soluble in acid medium, and the chitosan films obtained from such solutions retain this acid character, in which chitosan is present as chitosan ion. A film formed in this way, in contact with water or buffered physiological solutions (for example, phosphate buffer solution (PBS)) confers an acid character to said solution and the film quickly loses its integrity, falling a part in a short time period. Due to this phenomenon it is necessary to stabilize it, the normally used process consisting of the immersion of chitosan in a strongly basic solution, normally NaOH, for at least 1 hour. This basic character is one that may affect some of the supports commonly used in tissue engineering, for example, polylactic acid or polyglycolic acid.

The second drawback, perhaps the most important from the point of view of its practical application, lies in the low cell adherence if compared to that obtained in a chitosan film formed according to existing protocols with respect to that produced in the commercial plastics treated for this purpose. Furthermore, this is worsened by the lack of homogeneity of cell adherence. The cells proliferate only on certain areas of the film and drastically change their morphological form, which in turn implies an important alteration in the metabolism and specific characteristics thereof with respect to that which is considered normal for these cell lines.

Thus, above all, the low rate and this lack of uniformity in cell adherence make its use as a coating material difficult, either for prosthesis commonly used in medical-surgical and dental practice, or else for gauzes of biomaterials already used in tissue engineering, such as, for example, poly-L-lactic acid or polyanhydrides.

### SUMMARY OF THE INVENTION

The invention addresses the problem of providing chitosan films able to allow a good cell adhesion and proliferation, and which, in addition, can be activated through the incorporation of substances with biological activity, which would provide them with an important application in the medical-pharmaceutical field.

The solution provided by the present invention is based on the fact that the inventors have observed that carrying out a drying stage on a previously stabilized and washed chitosan based film considerably increases the capacity for cell adherence to the chitosan based film submitted to said treatment of drying. By means of this treatment, it is possible to obtain chitosan based films that show characteristics of cell adherence and proliferation similar to or greater than those obtained in commercial plastics (for example, plates of wells) treated for this purpose, as well as properties which allow them to immobilize, either through adsorption or covalently, substances with biological activity, including bone morphogenetic proteins (BMP), maintaining their biological activity.

Therefore, an object of this invention constitutes a method for the production of a chitosan based film , with increased cell adherence capacity. Said film constitutes an additional object of this invention.

Another additional object of this invention consists of a method for the production of a chitosan based film, with increased cell adherence capacity, biologically activated with a substance with biological activity. The resulting film constitutes another additional object of this invention.

Another additional object of this invention consists of a chitosan based film totally or partially coated product , such as, for example, an implant of dental or traumatologic use.

A further additional object of this invention consists of the applications of said chitosan based films, such as the use of said chitosan based film with increased cell adherence as a vehicle for the transport and release of substances with biological activity, or their use in the elaboration of a biologically activated chitosan based film with increased capacity for cell adherence. Said biologically activated chitosan based film may be used, in turn, in multiple applications, such as in the induction of biological activity in a receiving organism, in the enhancement of osteointegration of implants of dental or traumatologic use and/or in the regeneration of tissues, for example, osseous tissue, among other applications.

### BRIEF DESCRIPTION OF THE FIGURES

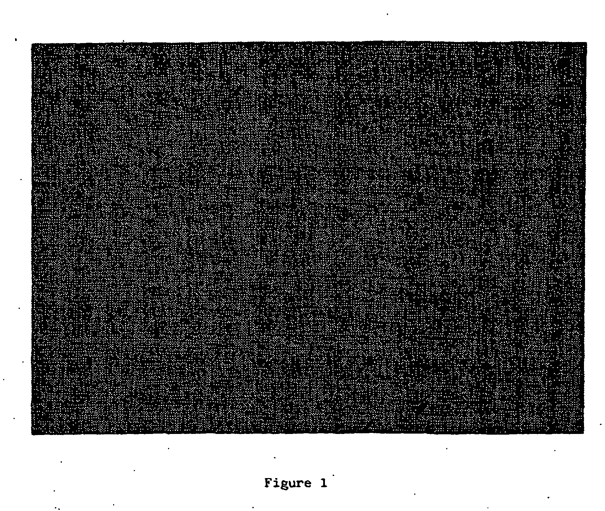

Figure 1 shows a photograph, obtained by inverse optical microscopy (100 x), of a cell culture C2C12 seeded over wells containing chitosan films stabilized with NaOH not submitted to treatment of activation of the cell adherence capacity according to the invention (Comparative Example), wherein the rounded appearance of the cells can be observed, indicating that they are in suspension.

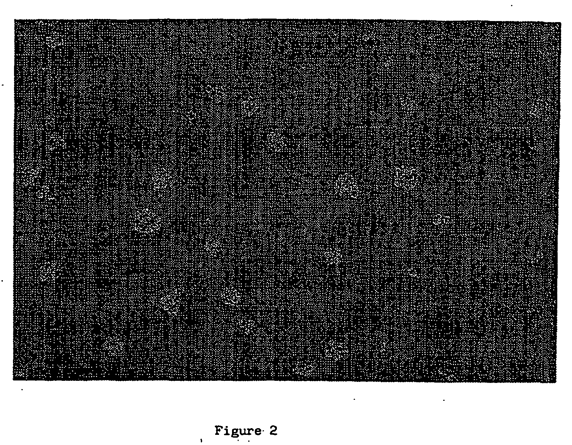

Figure 2 shows a photograph, obtained by inverse optical microscopy (100 x), of a cell culture C2C12 seeded over wells containing chitosan films stabilized with NaOH not submitted to treatment of activation of the cell adherence capacity according to the invention (Comparative Example), in an later stage than the one shown in Figure 1, namely, after changing the culture medium with the consequent dragging of cells not adhered to the film, in which the grouping of cells in the form of racemes can be seen, indicating an atypical growth pattern.

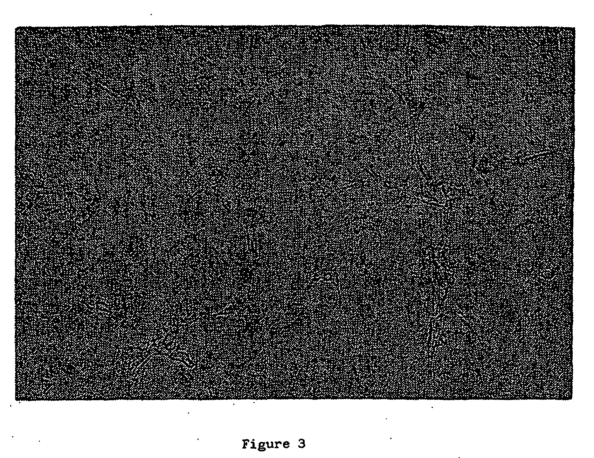

Figure 3 is a photograph, obtained by inverse optical microscopy (100 x), of a cell culture C2C12 seeded over wells containing chitosan films stabilized with glutaraldehyde and NaOH not submitted to treatment of activation of the cell adherence capacity according to the invention (Comparative Example), wherein an altered morphology of said cells can be seen due to their adhesion to the film, indicating an anomalous pattern of growth, as well as a modification of their cytoarchitecture.

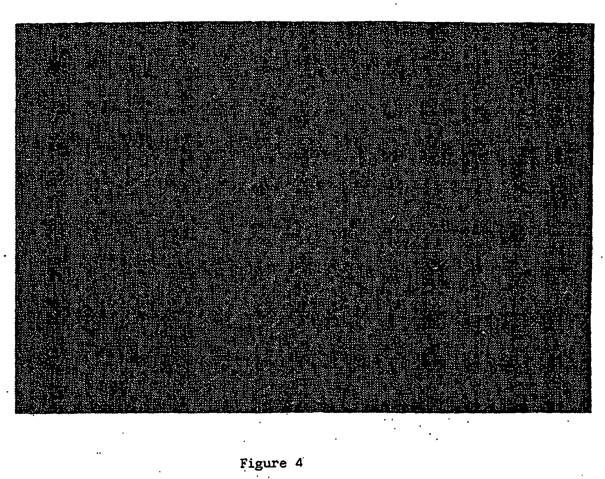

Figure 4 is a photograph, obtained by inverse optical microscopy (100 x), of a cell culture C2C12 seeded over wells containing chitosan films stabilized with NaOH submitted to treatment of activation of the cell adherence capacity according to the invention (Example 1), in which it can be seen that the entire surface of the film is completely covered with cells in the form of a monolayer until reaching confluence.

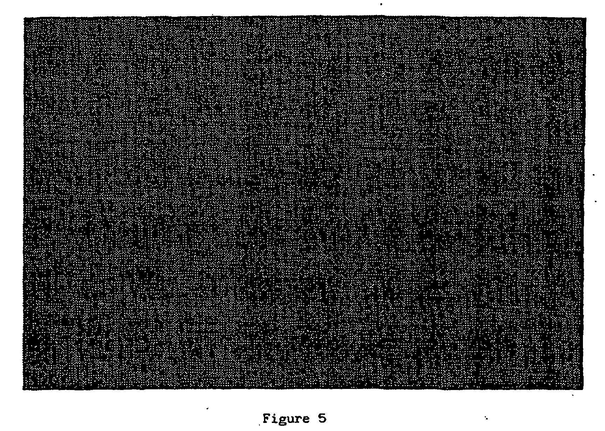

Figure 5 is a photograph, obtained by inverse optical microscopy (100 x), of a cell culture C2C12 seeded over wells containing chitosan films stabilized with NaOH submitted to treatment of activation of the cell adherence capacity according to the invention (Example 1), in a stage prior to the one shown in Figure 4, in which parts of film as yet uncoated by the cells can be seen.

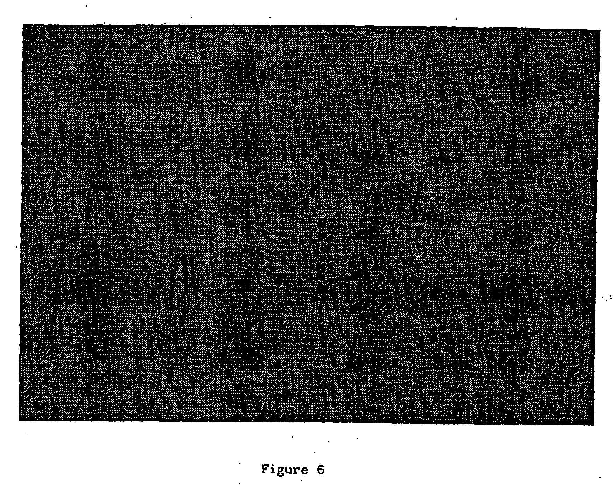

Figure 6 is a photograph, obtained by inverse optical microscopy (100 x), of a cell culture ROS 17/2.8 seeded over wells containing chitosan films stabilized with NaOH submitted to treatment of activation of the cell adherence capacity according to the invention (Example 1), wherein it can be seen that the entire surface of the film is completely covered with cells in the form of a monolayer until reached confluence.

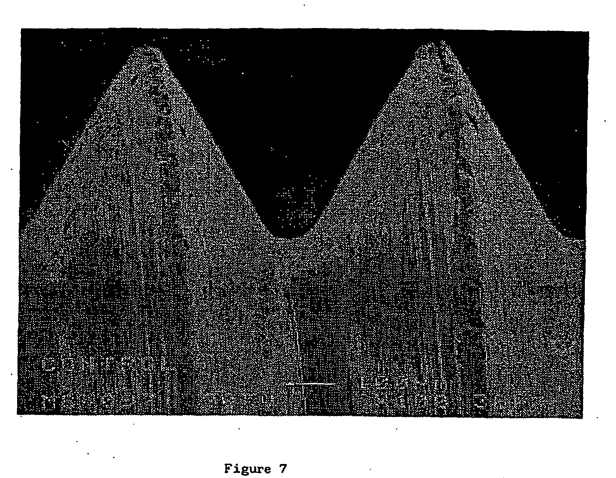

Figure 7 is a photograph (100x) obtained by scanning electron microscopy (SEM) of a fragment of a titanium screw used in the trials of implants in experimental animals, before being coated with a chitosan film, wherein the lines of mechanization of the screw can be seen.

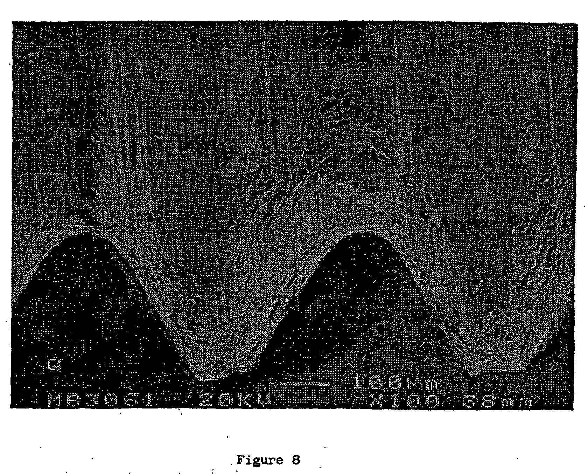

Figure 8 is a photograph (100x) obtained by scanning electron microscopy (SEM) of a fragment of a titanium screw used in the trials of implants in experimental animals, coated with a chitosan film stabilized with NaOH and submitted to a treatment of activation of the capacity of cell adherence provided by this invention.

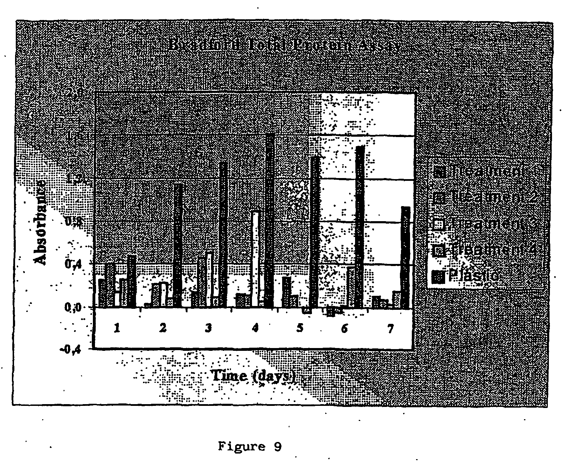

Figure 9 is a bar diagram illustrating the total protein content of the cell culture, determined by the Bradford method, with respect to the culture time (days), wherein the value of absorbency is indicative of the protein content of the cell culture in each case, and, indirectly, of the quantity of cells adhered to the film; the values obtained using a conventional well plate (plastic) used as reference are compared against the values obtained using chitosan films stabilized with different treatments of stabilization not submitted to activating treatment of the capacity of cell adherence provided by this invention. Treatment 1: stabilization with phosphate buffer; Treatment 2: stabilization with NaOH; Treatment 3: stabilization with glutaraldehyde and NaOH; and Treatment 4: stabilization with glutaraldehyde.

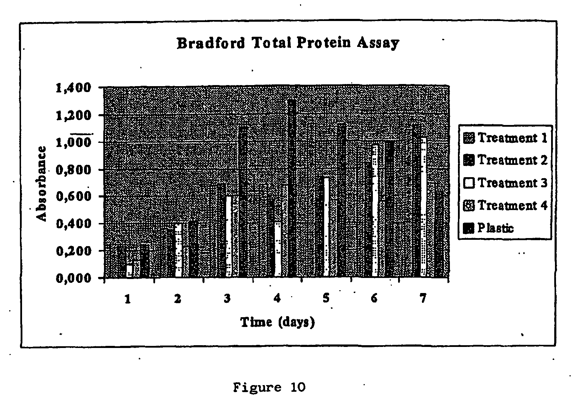

Figure 10 is a bar diagram illustrating the total protein content of the cell culture, determined by the Bradford method, with respect to the culture time (days), in which the value of absorbency is indicative of the protein content of the cell culture in each case, and, indirectly, of the quantity of cells adhered to the film; the values obtained using a conventional well plate (plastic) used as reference are compared against the values obtained using chitosan films stabilized with different treatments of stabilization subsequently submitted to treatment of activation of the capacity of cell adherence provided by this invention. Treatment 1: stabilization with phosphate buffer; Treatment 2: stabilization with NaOH; Treatment 3: stabilization with glutaraldehyde and NaOH; and Treatment 4: stabilization with glutaraldehyde.

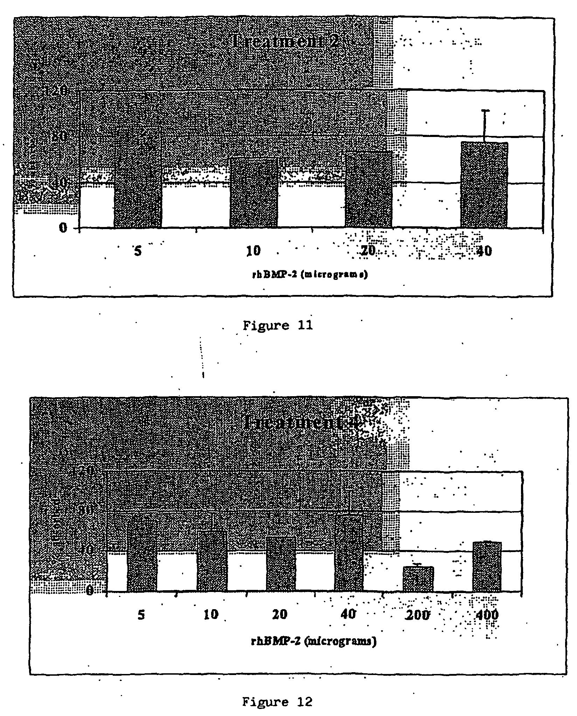

Figure 11 is a bar diagram illustrating the percentage of rhBMP-2 absorbed by chitosan films stabilized with NaOH and submitted to treatment of activation of the capacity of cell adherence provided by this invention against the quantity of protein added.
Figure 12 is a bar diagram illustrating the percentage of rhBMP-2 absorbed by chitosan films stabilized with glutaraldehyde and submitted to treatment of activation of the capacity of cell adherence provided by this invention against the quantity of protein added.

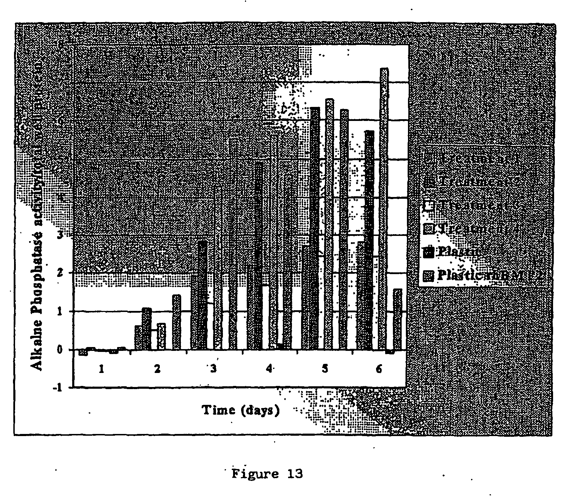

Figure 13 is a bar diagram illustrating total alkaline phosphatase/protein activity in the wells on different days; the results obtained show that rhBMP-2, bound to chitosan films stabilized through different treatments and submitted to treatment to activate the capacity of cell adherence provided by this invention, remains active; the values obtained are compared against those obtained using a conventional well plate (plastic) used as reference. Treatment 1: stabilization with phosphate buffer; Treatment 2: stabilization with NaOH; Treatment 3: stabilization with glutaraldehyde and NaOH; and Treatment 4: stabilization with glutaraldehyde.

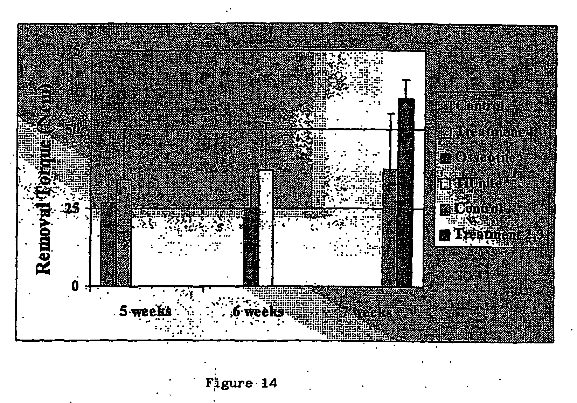

Figure 14 is a bar diagram showing the torque or force couple necessary to unscrew the previously implanted implant in the flat part of the tibia of rabbits used at different times; the results obtained using chitosan film coated titanium screws provided by this invention, stabilized by different treatments (NaOH: Treatment 2; Glutaraldehyde and NaOH: Treatment 3; and Glutaraldehyde: Treatment 4), submitted to treatment of activation of the capacity of cell adherence provided by this invention and biological activated with rhBMP-2, are compared against the results obtained with controls (no coated titanium screws); in addition, for purposes of comparison, the values obtained with commercial screws Osseotite® and TiUnite® (Nobel Pharma) are included [data taken from Gottlob et al., Applied Osteointegration Research, 2000, 1: 25-27].

### DETAILED DESCRIPTION OF THE INVENTION

The present invention relates, in general, to the production of chitosan based films with increased capacity for cell adherence, and that can be biologically activated, as well as the resulting films and their applications.

In general, the production method for chitosan based films with increased cell adherence provided by this invention comprises, in general, the general stages of formation of the chitosan based film, its stabilization and treatment of activation of the capacity of cell adherence of the stabilized film. On the other hand, the biological activation of the chitosan based film may be performed once the film has been formed or, alternatively, by incorporating the substance with biological activity into any of the stages in the film production process, for example during the dissolution of the chitosan solution or during stabilization of the film, depending on the stability and nature of the substances with biological activity to be used, as well as the processing undergone by the film in other stages.

More specifically, the invention provides a method for the production of a chitosan based film with increased capacity for cell adhesion, hereinafter the method of the invention, which comprises:
a) dissolving chitosan, optionally along with a biodegradable polymer, in a solubilization medium comprising an aqueous solution of an acid;
b) depositing the solution resulting from stage a) on a surface;
c) drying said solution deposited on said surface, in order to obtain a chitosan based film ;
d) bringing said chitosan based film into contact with a stabilization agent selected from (i) an aqueous solution of a base, (ii) a pH buffer equal to or greater than 5, (iii) a link-forming agent, and (iv) mixtures thereof
e) washing the stabilized chitosan based film obtained in stage d); and
f) drying said stabilized chitosan based film, at a temperature lying between 15°C and 80°C, in the presence of an air current.

The chitosan based film with increased capacity of cell adherence, obtainable using the method of the invention, is fully or partially comprised of chitosan. Chitosan is a natural polymer obtained through partial deacetyllation of chitin. Chitin can be obtained from many different sources, for example, crustaceans, fungi, etc. The origin of the chitin used for obtaining the chitosan to be used in the present invention is not important. Similarly, it is possible to carry out modifications to the chitosan in order to introduce different functional groups, which, for example, may favour *in vivo* biological degradation of chitosan, stimulate osseous regeneration, etc. By way of illustration, said functional groups include phosphonic groups, carboxymethyl groups, methylpyrrolidone groups, etc. In general, any modification to the chitosan can be performed, provided that the final product keeps its filmforming capacity. In a particular embodiment, the chitosan used in the present invention comprises chitosan derivatized with one or more functional groups, selected among phosphonic groups, carboxymethyl groups, methylpyrrolidone groups and mixtures thereof.

Chitosan, being a polymer obtained by partial deacetyllation of chitin, shows a very broad range of molecular weights and degrees of deacetyllation. These parameters depend on the specific conditions used in the basic hydrolysis performed on the starting material (chitin), as well as the origin thereof. Thus, it is possible to obtain, for example, chitosans whose average molecular weight lies between 150,000 and 2,000,000 and even greater, with degrees of deacetyllation lying between 65% and 95% or even greater. Any of said chitosans can be used in putting the present invention into practice, although those chitosans with medium and low average molecular weights are preferred, for example, between 200,000 and 500,000, and with medium and high degrees of deacetyllation, for example, between 65% and 95%.

The biodegradable polymer, in the case that it is used, may be any natural or synthetic polymer, provided that it is soluble in water or in aqueous acid medium and biodegradable. Illustrative examples of biodegradable polymers that can be used in the present invention include polyglycolic acid, alginate, carrageenate, collagen, etc, and mixtures thereof.

The chitosan solubilization medium, optionally along with the biodegradable polymer, is a medium comprising an aqueous solution of an organic or inorganic acid, with a pH equal to or less than 3.5.

In general, any acid can be used in which chitosan, and, if applicable, the biodegradable polymer, are soluble. By way of illustration, said acid can be hydrochloric acid, acetic acid, citric acid, lactic acid, malic acid, etc. In a particular embodiment, the solubilization medium is an aqueous solution of acetic acid.

The concentration of chitosan in the resulting solution may vary within a broad range, having reached concentrations of up to 5 % in chitosan (in other words, greater than those normally used in the formation of chitosan films).

The chitosan solution, optionally along with the biodegradable polymer, hereinafter, chitosan based solution, should have a viscosity appropriate for the application to which it is aimed. This viscosity may vary within a broad range, depending on whether said chitosan based solution is to be applied on a flat surface or on a complex surface, or whether it is to be used as a medium for coating an article by immersion, for example, a screw of the type used in dental implants.

The viscosity of the chitosan based solution is determined by several factors intrinsic to the solution itself. On one hand, by the concentration of chitosan, and on the other, by the average molecular weight of the chitosan used in the preparation of said solution, and, finally, by the solubilization medium used.

In general, the viscosity is not a determining factor in the practical embodiment of the present invention, except in the case of formation of films on complex surfaces, where this parameter has to be sufficiently high to allow the formation of a homogeneous film thereon during the process of evaporation of the solution.

In several practical embodiments of the present invention, 1 % solutions of chitosan in 50 mM acetic acid have been routinely used that possessed intrinsic viscosities that varied from 17 g.dL⁻¹ to 4 g.dL⁻¹, depending on the molecular weight of the chitosan used.

Once the chitosan based solution has been prepared with the appropriate viscosity, said solution is uniformly deposited or extended by conventional methods, for example, by spraying, immersion, application with brush, etc., on a surface, such as a flat or complex surface, or on the surface to be coated with a chitosan based film provided by this invention.

Once the chitosan based solution has been deposited on said surface, a drying is performed in order to remove the solvent, for example, by simple evaporation, obtaining a chitosan based film on said surface.

The drying can be performed by any conventional method. In general, it can be performed at a temperature lying between room temperature and a temperature in which thermal decomposition of the chitosan does not occur, for example, at a temperature lying between 15° C and 80° C, optionally in the presence of an air current, for an appropriate period of time until no weight change is observed. In general, increasing the temperature reduces the duration of this stage. When the chitosan based films are for applications related to cell growth and proliferation, it is useful to work in a sterile room or in a laminar flow chamber with an air current in order to obtain an uncontaminated film. In a particular embodiment, the drying of the chitosan based film is performed at a temperature lying between 20° C and 40° C, under an air current. In the conditions indicated, the drying stage may last between 12 and 24 hours.

The chitosan based film previously obtained cannot be used immediately because of its strongly acid character. As it was indicated earlier, said film in contact with water or with physiologically buffered solutions, such as PBS, quickly decomposes. Due to this reason, it is necessary to stabilize the film before use.

To stabilize the chitosan based film previously formed, said film is brought into contact with a stabilization agent, which may be (a) a neutralizing agent, such as an aqueous solution of a base or a pH buffer equal to or greater than 5; (b) a link-forming agent; or (c) mixtures thereof.

The procedure normally used for stabilizing the chitosan films consists of immersing the film (or the object or surface coated with it) in a strongly basic solution, generally 1 M NaOH for a minimum of 1 hour, at times for as long as 24 hours. However, said procedure may, as it has already been mentioned, negatively affect the stability of the film coated support. The effect of the treatment also produces, and this is precisely its purpose, a total neutralization of the charges present on the chitosan ions, thus giving rise to a strong reduction in interaction between the film and the film coated support.

Although it is possible to use the stabilization protocol previously mentioned, in the present invention it is preferred to use less drastic conditions in the basic treatment, reducing both the concentration of the base (NaOH) used and the time of exposure thereto. In a particular embodiment, an immersion time lying between 30 and 40 minutes in an aqueous solution of 0.5 M NaOH is used.

Alternatively, it is possible to use other gentler media, such as those provided by buffer solutions with a pH equal to or greater than 5, for example, carbonate or phosphate buffer solutions, to neutralize the charge of the chitosan film.

Another way of stabilizing the chitosan based film formed comprises the linking, by means of the use of link-forming agents, such as bi-functional reagents, between the chains of chitosan present in the film. In a particular embodiment, the linking agent used is glutaraldehyde as this is the commonly used bi-functional reagent. Glutaraldehyde can be used with strongly alkaline medium, such as that provided by NaOH, or else with a gentler medium, such as the one provided by the carbonate or phosphate buffer. In these cases, the stabilization of the film is achieved at the same time as the neutralization of its charges with the linking of the chitosan chains.

In accordance with the above, for the stabilization of chitosan based films previously obtained, it is possible to use, in a particular embodiment, a phosphate buffer of molarity lying between 0.1 and 1 M, preferably 0.25 M, at a value of pH close to neutrality. Glutaraldehyde can be used as a link-forming agent, at concentrations of up to 0.1 %, preferably less than 0.05 % and more preferably less than 0.025 % or less. Combinations of both stabilizing effects have also been used, for example, simultaneous treatment with NaOH and glutaraldehyde in concentrations of 0.5 M and 0.025 %, respectively. In another particular embodiment, a stabilization agent comprising a buffer, such as phosphate buffer or carbonate buffer, and glutaraldehyde was used.

The time of exposure to the different agents is variable, with exposures between 30 and 45 minutes being preferred, although longer times are possible.

Once the chitosan based film has been stabilized, the stabilization agent used for the purpose is withdrawn and it is washed several times, to eliminate the remains of the stabilization agent used, with sufficient PBS volume or any other medium compatible with the biological use proposed for said films.

The film thus stabilized is still not able to produce uniform and homogeneous cellular adherence over the entire surface. As it is shown in Comparative Example, if, after washing, seeding of established adherent cells lines is performed, the trials of the quantity of DNA present indicate that only approximately one third of the seeded cells adhere to the film according to the previously described method (Figure 1). This adherence is not homogeneous but instead occurs in certain areas of the film, with a large part of its surface without adhered cells (Figure 2). Furthermore, the cell proliferation produced in these adherent cells does not tend to produce expansion over the whole surface of the film, but rather it is localized around the initial point of adherence (Figure 2), altering the cellular cytoarchitecture and changing the typical morphology of the fibroblasts of these cell lines (Figure 3). This fact makes it impossible in practice to use said chitosan based films for guided tissue regeneration.

The aforementioned cellular adherence is very variable, depending on the origin of the chitosan, the chitosan production method used, the average molecular weight of the chitosan used, the time and conditions of storage of the chitosan film prior to stabilization, etc. There is no clear correlation between the different mentioned variables and homogeneous cell adhesion to the film, obtaining, as it was said at the beginning, very variable results.

The fact that a prolonged storage time (at times of months) of the chitosan based film appears to favorably affect cell adhesion with respect to the same film formed and used immediately (whereby the influence of the molecular weight of the chitosan used is eliminated, the weights of the laminas also being the same), seems to indicate that a slight structural change is produced that has a subtle effect on adherence.

None the less, the random nature of this phenomenon indicates the need to develop a procedure that homogenizes the behavior of the chitosan film with respect to the capacity for cell binding and proliferation, such that there is an "activation" of the chitosan in this sense. With this aim, the following stage of the method of the invention is performed, consisting of treatment of activation of the cell adherence capacity of the stabilized film.

During the development of the present invention it was surprisingly found that the embodiment that, at least, a second drying of the chitosan based film, already stabilized, and washing in the same conditions as those used in the initial formation thereof, homogenizes the behavior of the chitosan film with respect to cell adherence, obtaining cell densities after seeding similar to those presented by the same cells in bottles of commercially available cultures, with levels of enzymatic activity (indicating the viability of the culture) and quantity of DNA equivalent to those obtained in the latter of these. The cell proliferation is homogeneous, maintaining the typical microscopic cell morphology of the seeded lines.

The second drying is performed by any conventional method, at a temperature lying between 15° C and 80° C, in the presence of an air current, during a suitable period of time. The drying is performed until there is no weight change. In a particular embodiment, the drying of the chitosan based film is performed at a temperature lying between 20° C and 40° C, under an air current. In the conditions indicated, constant weight is usually attained in 12-24 hours.

The chitosan based film, which can be obtained by the method of the invention, has an increased cellular adherence capacity. The capacity of a cellular adherence film can be determined by different trials. By way of illustration, said capacity of cell adherence may be determined by using any adherent cell line, for example, C2C12 cells, by means of the trial denominated "Ethidium Homodimer Trial" [see Example 1 where the protocol for said trial is written] which, briefly, comprises cultivating C2C12 cells in chitosan based film coated wells or uncoated wells (plastic), lysing the cells, adding ethidium homodimer, incubating and reading the Fluorescence Intensity emitted at 645 nm after excitation at 530 nm. In the sense used in this description, the expression "chitosan based film with increased cell adherence capacity" refers to the fact that said chitosan based film has a cell adherence capacity greater than that which the same film has in normal conditions, that is, without having been previously submitted to any specific treatment to activate their capacity of cell adhesion. By way of illustration, the treatment of activation of the cell adherence capacity of this invention provides a chitosan based film with a cell adherence capacity activated by said treatment, determined by the Ethidium Homodimer Trial, equal to or greater than the increase of, at least, 25 %, preferably of, at least, 50 % in the value of the capacity of cell adherence determined by said Ethidium Homodimer Trial on a chitosan film not submitted to said treatment of activation of the cell adherence capacity.

The chitosan based film with increased cell adherence capacity, which can be obtained through the method of the invention, constitutes an additional object of this invention.

The chitosan based film with cell adhesion capacity may be used to adhere and grow cells. To do this, it may be useful to immobilize substances with capacity to stimulate cell proliferation on said film.

Additionally, the chitosan based film with increased capacity of cell adherence can be used as a vehicle of substances with biological activity so that it can fix or immobilize substances with biological activity and, optionally, release them in places of interest.

Therefore, the invention provides a biologically activated chitosan film with increased cell adherence capacity, which comprises said chitosan film that can be obtained according to the method of the invention and, at least, a substance of biological activity.

As a substance with biological activity, any substance of natural, synthetic or recombinant origin can be used that is able to exercise biological activity in a recipient organism, such as the human or animal body. By way of illustration, said substance with biological activity may be an antibiotic, an hormone, a protein, etc. In a particular embodiment, said substance with biological activity is a protein belonging to the bone morphogenetic proteins (BMP), such as a human, natural or recombinant BMP, or a dimer or heterodimer thereof, for example, a recombinant human BMP (rhBMP). In a particular embodiment, said rhBMP is selected from rhBMP-2, rhBMP-4, rhBMP-7, dimers or heterodimers thereof, and mixtures thereof.

The biologically activated chitosan film with increased cell adherence capacity can be obtained by a procedure comprising bringing a chitosan film with increased cell adherence capacity, that can be obtained according to the method of the invention, into contact with said substance with biological activity.

Alternatively, the biologically activated chitosan film with increased cell adherence capacity can be obtained by means of a procedure comprising bringing said substance with biological activity into contact either with a chitosan solution, optionally along with a biodegradable polymer, in a solubilization medium comprising an aqueous solution of an acid, in stage a) of the method of the invention, or else, alternatively, with the chitosan based film in contact with the stabilization agent, in stage d) of the method of the invention.

The treatment of biological activation of the stabilized, washed and dried chitosan based film has the aim of biologically activating said film to induce the desired biological activity in the recipient organism. Said induction is achieved through fixing the chitosan based film of said substance with biological activity. The fixing or immobilization of said substance with biological activity to the chitosan based film may be achieved either through direct adsorption of said substance on the film, or by covalent immobilization thereof on the film, for example, by interactions between reactive groups present in proteins with glutaraldehyde.

Example 2 describes the induction of alkaline phosphatase activity on the cell line C2C12 produced by rhBMP-2 adsorbed or covalently immobilized on the chitosan films. This induction compares favourably with that obtained in said cell line seeded over commercial plastic and treated with the same doses of rhBMP-2 in solution, doses present in the culture medium during the whole time of the trial. As it can be seen in said Example 2 (see Table V), there appears to be a synergic effect between chitosan and rh-BMP-2 in terms of its biological activity.

The invention also provides an article or a product coated with a chitosan based film, comprising a support and a total or partial coating of said support with a chitosan film with increased cell adherence capacity, optionally activated biologically. In a particular embodiment, said coated article or product is a prosthesis or a medical-surgical implant, for example, an implant of dental or traumatologic use and said chitosan based film is a biologically activated chitosan film comprising, at least, a BMP. In another particular embodiment, said support is selected from among gauzes and matrices of biocompatible and/or biodegradable polymers used in tissue engineering.

In another aspect, the invention relates to the use of a chitosan based biologically activated film with increased cell adhesion capacity, provided by this invention, in the induction of a biological activity in a recipient organism, in the enhancement of osteointegration of implants used in dental surgery or traumatologic surgery in the entirety or part of the zone of the recipient organisms where it is desired to enhance and/or in the regeneration of osseous tissue.

More specifically, the invention also relates to the use of a biologically activated chitosan film with increased cell adherence capacity provided by this invention, in the elaboration of an implant of dental or traumatologic use.

The invention also provides means for inducing biological activity in a recipient organism, wherein a support with a chitosan based biologically activated film with increased cell adherence capacity provided by this invention is to be implanted in said organism in need of said biological activity.

The invention also provides means for enhancing the osteointegration of implants of dental or traumatologic use in a recipient organism wherein an implant coated with a biologically activated film of chitosan with increased cell adherence capacity, provided by this invention wherein said substance with biological activity is a BMP is to be implanted in said recipient organism in need of enhancing the osteointegration of said implant.

The invention also provides means for osseous tissue regeneration in a recipient organism wherein a matrix of bone generation coated with a biologically activated chitosan film with increased cell adherence capacity, provided by this invention, is to be implanted in a recipient organism with need of regeneration of osseous tissue, wherein said substance with biological activity is a BMP.

As a result of this invention, as it has been expressed herein above in detail, it is possible to obtain, for the first time in the sector of the art that concerns us, chitosan based films capable of permitting a good cell adhesion and proliferation and, in addition, which can be activated by incorporation of substances with biological activity. Said chitosan based films constitute a biocompatible and biodegradable film, which is perfectly adaptable to the form of the object or implant to be coated and to which cells from the host organism can adhere.

In the case of three-dimensional porous matrices frequently used in tissue engineering, it allows the formation of a compound material that takes advantage of the mechanical properties of the support and that allows a suitable porosity to be maintained for the penetration and growth of surrounding tissue, as the chitosan does not block the pores formed in the body of the matrix because it is in the form of a film. At the same time, its cell adherence capacity along with the biological activation produced in all the material, both on the external surface and internal surface, allows the desired biological effect to be produced simultaneously throughout the body of the matrix and not through a penetration towards the interior. The activating agent used is available from the first moment to produce its effect on the whole of the support body.

These peculiar characteristics of the new chitosan based films provided by this invention make them particularly suitable for improving the osteointegration of implants and prosthesis used commonly in medical-surgical practice, by means of coating said materials with a chitosan based film and its activation by means of the incorporation of bone growth inducing factors, such as BMPs, of natural or recombinant origin, in dimeric or heterodimeric form.

Similarly, the chitosan based films of the present invention are also specially useful for the repair of osseous and osseouscartilaginous lesions based on coating gauzes or pieces of biocompatible and/or biodegradable polymers used commonly in tissue engineering, by means of total or partial coating thereof with a chitosan based film provided by this invention, activated by incorporation of BMPs and/or other substances with biological activity.

The following examples serve to illustrate the invention and should not be considered as limiting the scope thereof.

### Comparative Example

### Growth of cell lines on non-activated chitosan films for cell adhesion

A 1 % chitosan solution is prepared in 50 mM acetic acid. The solution is sterilized by filtration through 0.22 µm after undergoing a prefiltration through 0.45 µm.

200 µl of this solution are deposited in the wells of a 48-well plate, leaving some of them free to act as a control of commercially available plastic.

The plate is dried under an air current in a laminar flow chamber for one night at a temperature of 30° C. Once dry, the wells are treated in triplicate with 400 µl of some of the following stabilization agents for a period of time of 30 to 45 minutes: (i) 0.5 M NaOH; 0.25 M phosphate; (ii) 0.025 % glutaraldehyde; and (iii) a solution of 0.5 M NaOH and 0.025 % glutaraldehyde.

After the treatment period has elapsed, the medium is withdrawn and the plates are washed four times with 400 µl of PBS, leaving the medium in contact with the plates for 10 minutes with the film between washings.

Finally, the PBS is withdrawn and 200 µl of cell culture medium (high glucose DMEM, with penicillin/streptomycin) are added, and the wells are seeded with C2C12 or ROS cells at a density of 10,000 cells/cm². The medium is finally completed with a further 200 µl of culture medium and the wells are incubated at 37° C in a CO₂ oven. On the following day, the medium is withdrawn and the pertinent trials are performed on the different wells.

The results obtained, once the corresponding values for blanks for each condition have been subtracted, are presented in Tables I and II for the cell lines C2C12 and ROS, respectively. In all cases, unless indicated otherwise, the values in brackets indicate the percentage ratio between the value obtained in each case and the corresponding value obtained with a conventional well plate (plastic) used as reference.

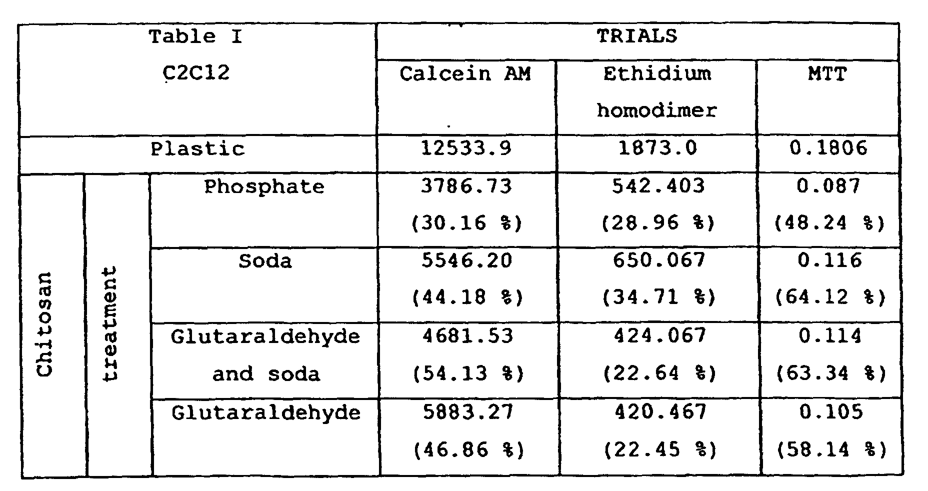

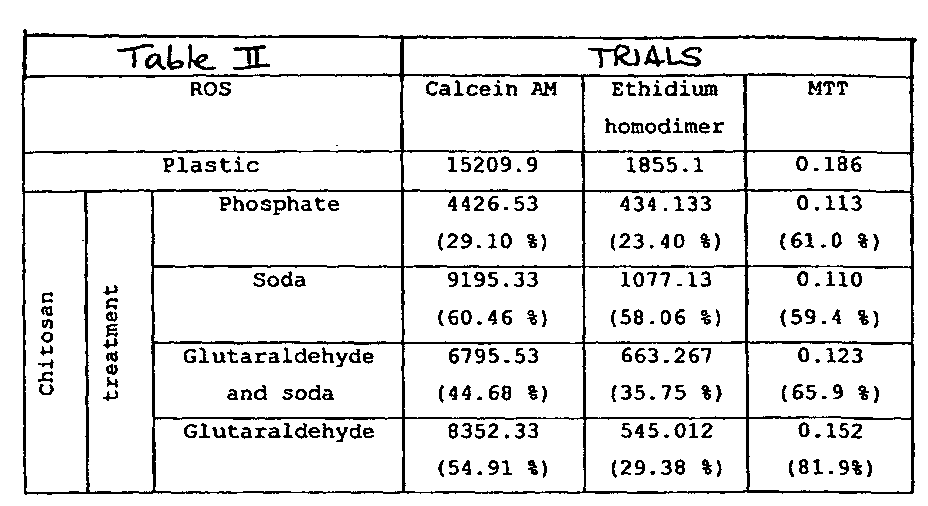

The protocols followed for performing the different trials are the following.

Calcein AM: The medium is withdrawn and the plates are washed with 200 µl of PBS. The PBS is withdrawn and 100 µl of calcein AM solution are added per well (obtained from the mixture of 10 µl of stock of calcein AM (4 mM) with 10 ml of PBS). The plate is incubated for 1 hour at room temperature in darkness and the Fluorescence Intensity emitted at 530 nm is read following excitation at 490 nm.

Ethidium homodimer: The calcein AM is withdrawn and the cells are killed by adding methanol at 70 %. After cell death, the methanol is withdrawn and 100 µl per well of ethidium homodimer solution are added per well (obtained from the addition of 20 µl of stock solution of ethidium homodimer (2 mM) to 10 ml of PBS). After incubation for 30 minutes, the Fluorescence Intensity emitted at 645 nm is read following excitation at 530 nm.

MTT: 40 µl (1/10 of volume existing in the well) of a solution formed by the reconstitution of 15 mg of MTT in 3 ml of PBS are added to the culture medium. The mixture is incubated for 2 hours at 37° C in a CO₂ oven. An equal volume of solubilization solution (basically Triton X-100 in isopropanol) is added to each well after that 2 hours, and after dissolution by repeated pipetting of the crystals of formazan formed, the optical density (OD) is read at 570 nm and at 690 nm, according to the protocol provided by the supplier of this trial kit (Sigma Chemical Company).

On the other hand, observation by inverse optical microscopy (lens: 10x and binocular: 10x, total 100x) of the culture cells of C2C12 and ROS showed that only approximately one third of the seeded cells adhere to the film formed according to the method described (Figure 1) and that the adherence is not homogeneous but that it is produced in certain areas of the film, there being a large part of the surface without adhered cells. In addition, it is also seen that the cell proliferation in these adherent cells does not tend to lead to expansion over the entire film, but rather, it is localized around the initial point of adherence (Figure 2), altering the cell cytoarchitecture and changing the typical morphology of fibroblasts of these cell lines (Figure 3). This fact makes the practical use of said chitosan based films impossible in guided tissue regeneration. Therefore, the chitosan film obtained and stabilized according to the protocol described in this Example is not able to show a uniform and homogeneous cell adhesion over the whole surface.

### Example 1

### Growth of cell lines on activated chitosan films for cell adhesion

The procedure described in Comparative Example is followed.

After washing with PBS, the medium is withdrawn and a second drying of the film is performed at 30-35° C under an air current.

Once dry, 200 µl of culture medium (DMEM high in glucose) are added, the cells are seeded at the same density as in Comparative Example and the wells completed with an additional 200 µl of medium. The plates are finally incubated in a CO₂ oven at 37° C. On the following day the same analyses as those described in Comparative Example are performed on the plates, following the protocols already described. The results obtained for the cell lines C2C12 and ROS are presented in Tables III and IV, respectively.

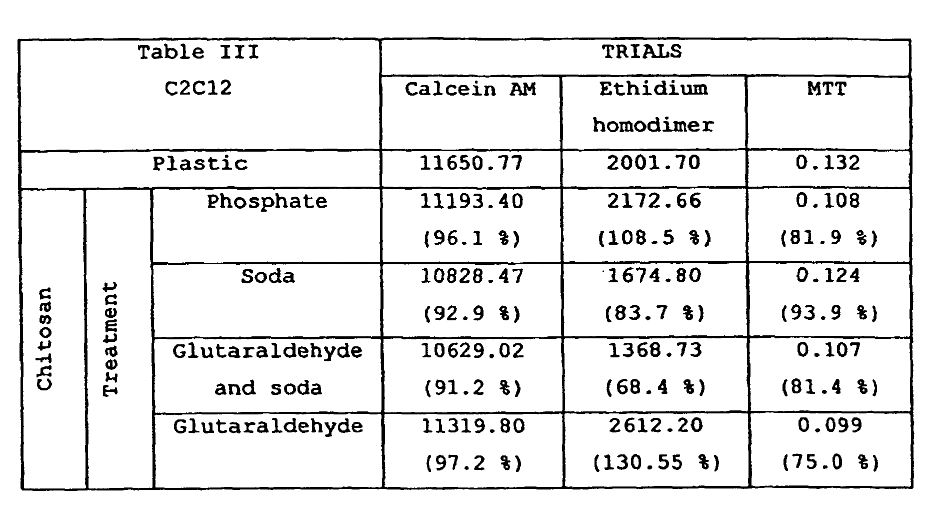

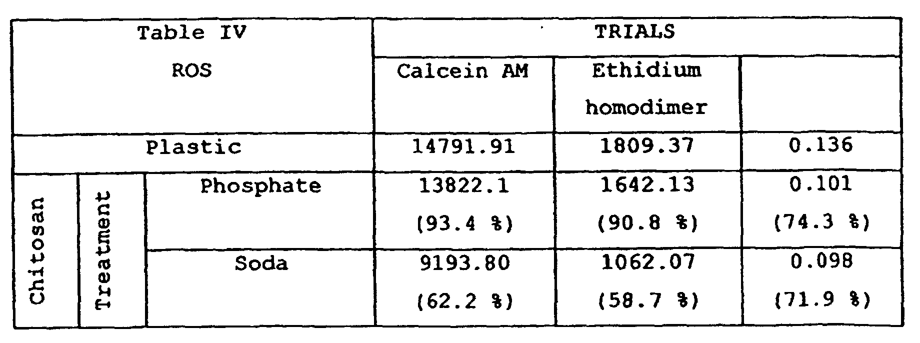

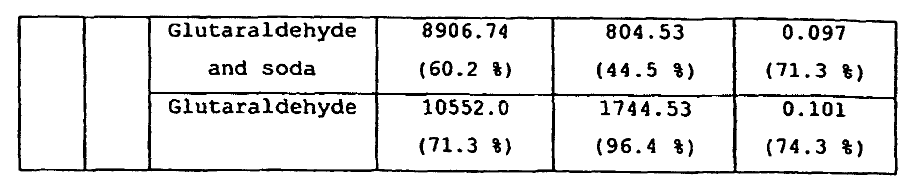

On comparing the values shown in Tables III and IV with those shown in Tables I and II, it is seen that the growth in cell lines assayed on the chitosan films submitted to treatment of activation of the cell adherence capacity (Example 1) is much greater than that obtained on the chitosan films not submitted to said treatment of activation of_the cell adherence capacity Comparative (Example).

Similarly, the observation by inverse optical microscopy (100 x) of the cell cultures of C2C12 and ROS on chitosan films submitted to different treatments of stabilization and treatment of activation of the cell adherence capacity showed that the seeded cells adhere sufficiently to the film formed according to the method described and that the cell proliferation in said adherent cells tends to lead to their expansion over the surface of the film, not altering the cellular architecture (Figures 4-6). This fact makes the practical use of chitosan based films possible for guided tissue regeneration. Therefore, the chitosan film obtained, stabilized and activated in terms of its cell adherence capacity, according to the protocol described in this Example, is able to show a uniform and homogeneous cell adherence over the whole surface.

### Example 2

### Activation of chitosan films by rhBMP-2

A solution formed of 40 µl of rhBMP-2 (at a concentration of 1 mg/ml in 50 mM acetic acid) is deposited on films prepared according to the protocol described in Example 1, after a second drying. There then follows a dilution with 160 µl of PBS. The wells are kept at 4° C overnight, then the medium with the protein is withdrawn the following day. The wells are then seeded with C2C12 according to the protocols described in Comparative Example or in Example 1, with an initial cell density of 20,000 cells/cm³.

40 µl of rhBMP-2 at a concentration of 1 mg/ml are added to the plastic control wells after seeding, while no addition of rhBMP-2 is made to the cells seeded on the chitosan films.

On the third day, the culture medium is withdrawn and changed for an equal volume of fresh medium, performing a new addition of rhBMP-2 on the control cells. No addition of rhBMP-2 is made to the cells seeded on the chitosan films during the entire trial, such that the activation produced has to be performed by rhBMP-2 adsorbed on the film prior to cell seeding.

In Table V the results obtained are collected for the alkaline phosphatase activity induced by rhBMP-2.

The protocol followed by carrying out this trial is as follows. The culture medium is withdrawn and washed once with 200 µl of PBS. The PBS is withdrawn and 100 µl per well of the lysis solution (Triton X-100 at 0.1 %, 50 mM Tris · HCl pH 6.8 and 10 mM MgCl₂) are added. The solution is frozen/defrosted to -80° C three times. Finally, 15 µl of this lysis solution is withdrawn from each well and 150 µl of a 1:1 solution of alkaline phosphatase and substrate are added (Sigma Chemical Company), with pre-heating to 37° C and preparation immediately before use. The solution is incubated for 10 minutes at 37° C and the reaction stopped 10 minutes following the addition of 150 µl of 0.5 M NaOH per well. Finally, the OD is read at 405 nm.

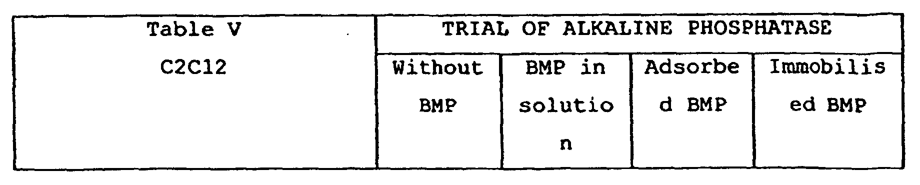

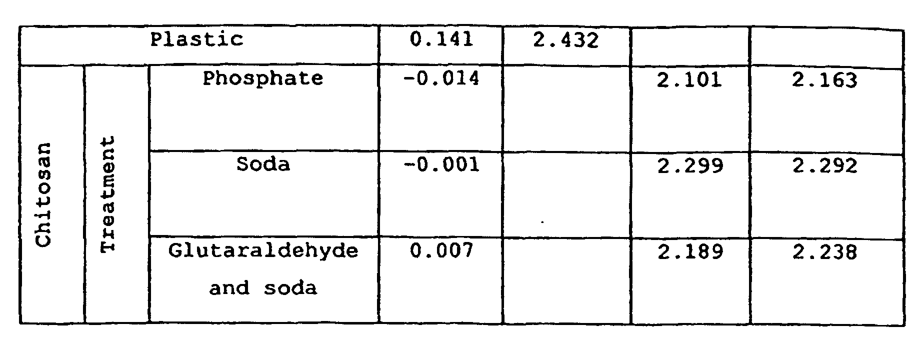

### Example 3

### Coating of an implant screw

A titanium screw (Figure 7) is submerged in a solution of 1 % chitosan in 50 mM acetic acid. It is then withdrawn and dried under an air current, keeping the screw in constant rotation, such that the film extends uniformly over the entire surface (Figure 8).

Once the film has been formed, it is treated according to one of the procedures described in Example 1, and activated according to that described in Example 2. It is then implanted in the proximal third of the internal face of the flat part of the tibia of rabbits of New Zealand breed, weighing 2.5 kg, supplied by the animal husbandry unit of the Universidad Complutense of Madrid (UCM). After three weeks, the animals are sacrificed, observing that the attachment is more stable than in the controls (titanium screws with no coating), needing forces of between 20-60 Newtons to unscrew the screws.

### Example 4

### Determination of the total protein content of cell culture

This trial was performed to determine the total protein content of the cell culture by means of the Bradford method, with respect to the culture time (days). In this trial, the value of absorbance is indicative of the protein content of the cell culture and, therefore, of the number of cells adhered to the film. The values that are obtained using a conventional well plate (plastic) used as reference are compared to the values obtained using chitosan films stabilized with different treatments of stabilization not submitted to treatment of activation of the cell adherence capacity provided by this invention (Example 4.1) and against the values obtained using chitosan films stabilized with different stabilization treatments submitted to treatment of activation of the cell adherence capacity provided by this invention (Example 4.2).

### 4.1 Chitosan films without activation of the cell adhesion capacity

In order to carry out this trial, 7 plates of 48 wells (Costar) were prepared as described below. Each plate had some wells containing different chitosan films (1 cm³) in triplicate, obtained by the process described in Example 1, stabilized by means of different treatments [Treatment 1: phosphate buffer; Treatment 2: NaOH; Treatment 3: glutaraldehyde and NaOH; and Treatment 4: glutaraldehyde] not submitted to treatment of activation of the cell adhesion capacity. Wells with film but without cells, wells without film, wells without cells and wells with cells were used as controls and blanks.

Then, 200 µl of cell culture medium (DMEM high in glucose with penicillin/streptomycin) are added to each well. The wells were seeded with C2C12 or ROS cells at a density of 10,000 cells/cm². Each well is completed with a further 200 µl of culture medium and incubated at 37° C in a CO₂ oven for the established period of time (1-7 days).

In order to carry out the trial for determining the total proteins of the cell culture by the Bradford method, the following method is followed. On the day of the trial, in a first instance, the culture medium is withdrawn and, then, the wells are washed with 400 µl of PBS (twice) to eliminate the remains of proteins that may have been present in the medium and which could have interfered with the trial. 200 µl of Bradford reagent (BioRad) per well are added along with 800 µl of distilled water or milliQ. The plates are incubated for 30 minutes in darkness at room temperature and the absorbance read at 595 nm.

The results obtained, once the values for the blanks corresponding to each condition have been subtracted, are shown in Figure 9, where the randomness of the values obtained can be appreciated, observing, as expected, that the cells have adhered to the plastic more efficiently than to the different chitosan films, where cell adhesion is non-existent or almost non-existent from the fourth day (coinciding with change of culture medium and the subsequent withdrawal of non-adhered cells) as it is shown by the drastic reduction in the values for absorbance measured from the fourth day onwards.

### 4.2 Chitosan films with activation of the cell adhesion capacity

The procedure described in Example 4.1 was repeated exactly, but replacing the chitosan films used in said example with chitosan films stabilized by different treatments [Treatment 1: phosphate buffer; Treatment 2: NaOH; Treatment 3: glutaraldehyde and NaOH; and Treatment 4: glutaraldehyde] and submitted to the treatment of activation of the cell adherence capacity of the invention described in Example 1.

The results obtained, once the values of the blank corresponding to each condition have been subtracted, are shown in Figure 10, where it can be appreciated that the cells have adhered to the chitosan films used in this case at levels similar or even greater than those of the plastic, even after the fourth day.

On comparing the values shown in Figures 9 and 10, it is observed that the adherence capacity and growth of the cell lines assayed on the chitosan films submitted to treatment of activation of the cell adherence capacity is very much greater than that obtained on chitosan films not submitted to said treatment of activation of the cell adherence capacity.

### Example 5

### Adsorption of rhBMP-2

An appropriate volume of a solution of rhBMP-2 (at a concentration of 1 mg/ml in 50 mM acetic acid) to obtain the desired quantity of protein in the well (5-400 µg of rhBMP-2) is added to films prepared according to the protocol described in Example 1, deposited in sections of 1 cm³ on the wells of a 48-well plate, making up the volume up to 200 µl with PBS when the volume of protein solution added to the well was less than 200 µl. Then, the plate is covered and kept in a cold chamber at 4° C overnight. On the following day, the protein solution is withdrawn from the wells (supernatant) and the total protein content in the supernatant is determined by the Bradford method (Example 4). By calculating the difference with the values obtained with the stock solution of protein, the quantity of protein adsorbed on the chitosan film is obtained.

The results of the adsorption of rhBMP-2 by chitosan films stabilized with NaOH, or with glutaraldehyde, and submitted to treatment of activation of the cell adherence capacity provided by this invention are shown in Figures 11 and 12, respectively, where the error bar corresponds to the mean of several experiments.

### Example 6

### Activation of chitosan films by rhBMP-2 over the course of time

This trial was performed to evaluate the capacity of activation of chitosan films by means of rhBMP-2 over the course of time. To do this, 10 µg of rhBMP-2 (from a solution of rhBMP-2 at a concentration of 1 mg/ml in 50 mM acetic acid) are added to films prepared according to the protocol described in Example 1, deposited in wells of a 48-well plate. The wells not coated with a film were used as controls.

The plates are kept at 4° C overnight, and on the following day, the medium with the protein is withdrawn. Then, the culture medium is added and the seeding with C2C12 is performed according to the protocols described in Comparative Example or in Example 1, with an initial cell density of 10,000 cells/cm².

After seeding, 10 µg of rhBMP-2 are added to the plastic control wells, while there is no subsequent addition of rhBMP-2 to the cells seeded over chitosan films.

On the fourth day, the culture medium is withdrawn and changed for an equal volume of fresh medium, performing a new addition of rhBMP-2 to the control cells. No addition of any rhBMP-2 is made during the whole trial to the cells seeded on chitosan films, such that the activation produced has to be performed by rhBMP-2 adsorbed on the film prior to cell seeding.

Figure 13 shows the results obtained for alkaline phosphatase activity (see the protocol of the trial described in Example 2) induced by total rhBMP-2 in wells on different days. The results obtained show that rhBMP-2, bound to the chitosan films stabilized by diverse treatments [Treatment 1: phosphate buffer; Treatment 2: NaOH; Treatment 3: glutaraldehyde and NaOH; and Treatment 4: glutaraldehyde] and submitted to treatment of activation of the cell adherence capacity provided by this invention, remains active during the time considered.

### Example 7

### Determination of the torque necessary to unscrew an implant

Following the procedure described in Example 3, titanium screws coated with chitosan films stabilized by various treatments [Treatment 2: NaOH; Treatment 3: glutaraldehyde and NaOH; and Treatment 4: glutaraldehyde] are prepared. Once the films have been formed on the screws, the cell adherence capacity was activated biologically by adsorption of rhBMP-2 according to the procedure described in Example 2. Uncoated titanium screws were used as controls.

Then, a screw coated with a chitosan film was implanted in the proximal third of the internal face of one of the flat part of the tibia of rabbits of the breed New Zealand, weighing 2.5 kg, supplied by the animal husbandry unit of the Universidad Complutense of Madrid [UCM] and the control screw was implanted in the flat part of the other tibia of the same animal. The implantation is performed using conventional methods. The animals are kept without any restriction on movement and, after the indicated time (5-7 weeks), the animals are sacrificed to evaluate the osteointegration of the implant by determining the torque needed to unscrew the screws.

The results obtained are shown in Figure 14 and show a more stable attachment in the case of chitosan film coated screws than in controls (uncoated titanium screws). In addition, the value of torque obtained with commercial screws Osseotite® and TiUnite® (Nobel Pharma) (data taken from Gottlob et al., Applied Osteointegration Research, 2000, 1:25-27) are provided by way of comparison.

## Claims

1. A method for producing a chitosan based film with increased cell adherence capacity, which comprises:
a) dissolving chitosan, optionally along with a biodegradable polymer, in a solubilization medium that comprises an aqueous solution of an acid;
b) depositing the solution resulting from stage a) on a surface;
c) drying said solution deposited on said surface, in order to obtain a chitosan based film;
d) bringing said chitosan based film into contact with a stabilization agent selected from (i) an aqueous solution of a base, (ii) a pH buffer equal to or greater than 5, (iii) an link-forming agent, and (iv) mixtures thereof;
e) washing the stabilized chitosan based film, obtained in stage d); and
f) drying said stabilized chitosan based film at a temperature lying between 15°C and 80°C, in the presence of an air current.

2. Method according to claim 1, wherein said chitosan has a mean molecular weight lying between 150,000 and 2,000,000, preferably between 200,000 and 500,000.

3. Method according to claim 1 or 2, wherein said chitosan has a degree of deacetyllation lying between 65% and 95%.

4. Method according to claim 1, wherein said chitosan comprises chitosan derivatized with one or more functional groups selected from phosphonic, carboxymethyl, methylpyrrolidone groups, and mixtures thereof.

5. Method according to claim 1, wherein said solubilization medium comprises an aqueous solution of an organic or inorganic acid, with a pH equal to or less than 3.5.

6. Method according to claim 5, wherein said acid is selected from hydrochloric acid, acetic acid, citric acid, lactic acid and malic acid.

7. Method according to claim 1, wherein chitosan is dissolved along with a biodegradable polymer.

8. Method according to claim 7, wherein said biodegradable polymer is selected from polyglycolic acid, alginate, carrageenate, collagen and mixtures thereof.

9. Method according to claim 1, wherein said stabilization agent comprises an aqueous solution of sodium hydroxide, phosphate buffer, carbonate buffer or glutaraldehyde.

10. Method according to claim 9, wherein said stabilization agent comprises an aqueous solution of sodium hydroxide and glutaraldehyde.

11. Method according to claim 9, wherein said stabilization agent comprises (i) a buffer selected from phosphate and carbonate buffer and (ii) glutaraldehyde.

12. Method according to claim 9, wherein the drying of the stabilized chitosan film in stage f) is performed at a temperature lying between 20° C and 40° C, in the presence of an air current.

13. A chitosan based film with increased cell adherence capacity, obtainable according to the method of anyone of claims 1 to 12, said chitosan based film with increased cell adherence capacity having a cell adherence capacity which is equal to or greater than the increase of, at least, 25% in the value of the cell adherence capacity over a chitosan film not submitted to a cell adherence capacity activation treatment, said cell adherence capacity being determined by the Ethidium Homodimer Trial.

14. A biologically activated chitosan based film with increased cell adherence capacity, comprising a chitosan based film with increased cell adherence capacity according to claim 13, and, at least, a substance with biological activity.

15. Biologically activated chitosan based film according to claim 14, wherein said substance with biological activity is selected from the group consisting of antibiotics, hormones, and proteins with biological activity in the human or animal body.

16. Biologically activated chitosan based film according to claim 15, wherein said substance with biological activity is a bone morphogenetic protein (BMP).

17. Biologically activated chitosan film according to claim 16, wherein said BMP is a human, natural or recombinant BMP, or a dimer or heterodimer thereof.

18. Biologically activated chitosan film according to claim 18, wherein said BMP is selected from among rhBMP-2, rhBMP-4, rhBMP-7, their dimers or heterodimers, and mixtures thereof.

19. A method for producing a biologically activated chitosan film with increased cell adherence capacity according to anyone of claims 14 to 18, which comprises bringing a chitosan film according to claim 13, or obtainable according to the method of anyone of claims 1 to 12, into contact with a substance with biological activity.

20. Method for producing a biologically activated chitosan film with increased cell adherence capacity according to anyone of claims 14 to 18, which comprises bringing a substance of biological activity into contact with (i) said chitosan solution, optionally along with a biodegradable polymer, in a solubilization medium that comprises an aqueous solution of an acid, in stage a) of the method of claim 1, or, alternatively, with (ii) the chitosan based film into contact with the stabilization agent, in stage d) of the method of claim 1.

21. A chitosan based film coated product comprising a support and a total or partial coating of said support with a chitosan film according to anyone of claims 13 to 18.

22. Product according to claim 21, wherein said support is selected from the group consisting of prosthesis and medical-surgical implants.

23. Product according to claim 21, wherein said support is a dental or traumatologic implant and said chitosan based film is a biologically activated chitosan film comprising, at least, one BMP.

24. Product according to claim 21, wherein said support is selected from the group consisting of gauzes and matrices of biocompatible and/or biodegradable polymers used in tissue engineering.

25. Use of a biologically activated chitosan film according to anyone of claims 14 to 18, in the elaboration of a product coated with said film for the induction of biological activity in the recipient organism, in the enhancement of osteointegration of implants of dental or traumatologic use in their entirety or in part in the area of the recipient organism where it is desired to enhance and/or the regeneration of osseous tissue.

26. Use of a biologically activated chitosan film according to anyone of claims 14 to 18, in the elaboration of an implant for dental or traumatologic use.

## Patentansprüche

1. Verfahren zum Herstellen eines Films auf Chitosanbasis mit einer erhöhten Zellhaftfähigkeit, wobei das Verfahren folgende Schritte umfaßt:
a) Auflösen von Chitosan, optional zusammen mit einem biologisch abbaubaren Polymer, in einem Solubilisierungsmedium, das eine wäßrige Lösung von einer Säure umfaßt;
b) Aufbringen der aus Stufe a) resultierenden Lösung auf eine Oberfläche;
c) Trocknen der auf die Oberfläche aufgebrachten Lösung, um einen Film auf Chitosanbasis zu erhalten;
d) den Film auf Chitosanbasis mit einem Stabilisierungsmittel in Kontakt bringen, das aus i) einer wäßrigen Lösung von einer Base, (ii) einem pH-Puffer gleich oder größer 5, (iii) einem Verknüpfungsmittel und (iv) Mischungen aus denselben ausgewählt ist;
e) Waschen des stabilisierten Films auf Chitosanbasis, der in Stufe d) erhalten wurde; und
f) Trocknen des stabilisierten Films auf Chitosanbasis bei einer Temperatur, die zwischen 15° C und 80° C liegt, in der Gegenwart eines Luftstroms.

2. Verfahren nach Anspruch 1, wobei das Chitosan ein mittleres Molekulargewicht umfaßt, das zwischen 150.000 und 2.000.000, bevorzugt zwischen 200.000 und 500.000 liegt.

3. Verfahren nach Anspruch 1 oder 2, wobei das Chitosan einen Deacetylierungsgrad umfaßt, der zwischen 65 % und 95 % liegt.

4. Verfahren nach Anspruch 1, wobei das Chitosan ein Chitosan umfaßt, das mit einer oder mehreren funktionellen Gruppen derivatisiert wurde, die aus Phosphon-, Carboxymethyl-, Methylpyrrolidon-Gruppen und deren Mischungen ausgewählt sind.

5. Verfahren nach Anspruch 1, wobei das Solubilisierungsmedium eine wäßrige Lösung von einer organischen oder anorganischen Säure mit einem pH gleich oder kleiner 3,5 umfaßt.

6. Verfahren nach Anspruch 5, wobei die Säure aus Chlorwasserstoffsäure, Essigsäure, Zitronensäure, Milchsäure und Maleinsäure ausgewählt ist.

7. Verfahren nach Anspruch 1, wobei ein Chitosan zusammen mit einem biologisch abbaubaren Polymer aufgelöst wird.

8. Verfahren nach Anspruch 7, wobei das biologisch abbaubare Polymer aus Polyglycolsäure, Alginat, Carrageen, Collagen und deren Mischungen ausgewählt ist.

9. Verfahren nach Anspruch 1, wobei das Stabilisierungsmittel eine wäßrige Lösung von Natriumhydroxid, Phosphatpuffer, Carbonatpuffer oder Glutaraldehyd umfaßt.

10. Verfahren nach Anspruch 9, wobei das Stabilisierungsmittel eine wäßrige Lösung aus Natriumhydroxid und Glutaraldehyd umfaßt.

11. Verfahren nach Anspruch 9, wobei das Stabilisierungsmittel (i) einen Puffer, der aus Phosphat und Carbonatpuffer ausgewählt ist, und (ii) Glutaraldehyd umfaßt.

12. Verfahren nach Anspruch 9, wobei das Trocknen des stabilisierten Chitosanfilms in einer Stufe f) bei einer Temperatur, die zwischen 20° C und 40° C liegt, in der Gegenwart eines Luftstroms ausgeführt wird.

13. Film auf Chitosanbasis mit einer erhöhten Zellhaftfähigkeit, der gemäß dem Verfahren nach einem der Ansprüche 1 bis 12 erhalten werden kann, wobei der Film auf Chitosanbasis mit einer erhöhten Zellhaftfähigkeit eine Zellhaftfähigkeit umfaßt, die gleich oder größer als der Anstieg von zumindest 25 % des Werts der Zellhaftfähigkeit gegenüber einem Chitosanfilm ist, der keiner Zellhaftfähigkeits-Aktivierungsbehandlung unterzogen wurde, wobei die Zellhaftfähigkeit durch den Ethidium-Homodimer-Versuch bestimmt wird.

14. Biologisch aktivierter Film auf Chitosanbasis mit einer erhöhten Zellhaftfähigkeit, der einen Film auf Chitosanbasis mit erhöhter Zellhaftfähigkeit nach Anspruch 13 und zumindest eine biologisch aktive Substanz umfaßt.

15. Biologisch aktivierter Film auf Chitosanbasis nach Anspruch14, wobei die biologisch aktive Substanz aus der Gruppe bestehend aus Antibiotika, Hormonen und Proteinen mit einer biologischen Aktivität im Körper von Menschen und Tieren ausgewählt ist.

16. Biologisch aktivierter Film auf Chitosanbasis nach Anspruch 15, wobei die biologisch aktive Substanz ein knochenbildendes Protein (BMP) ist.

17. Biologisch aktivierter Chitosanfilm nach Anspruch 16, wobei das BMP ein menschliches, natürliches oder rekombiniertes BMP oder dessen Dimer oder Heterodimer ist.

18. Biologisch aktivierter Chitosanfilm nach Anspruch 18, wobei das BMP aus rhBMP-2, rhBMP-4, rhBMP-7, ihren Dimeren oder Heterodimeren und deren Mischungen ausgewählt ist.

19. Verfahren zum Herstellen eines biologisch aktivierten Chitosanfilms mit einer erhöhten Zellhaftfähigkeit nach einem der Ansprüche 14 bis 18, das einen Schritt umfaßt, in dem ein Chitosanfilm nach Anspruch 13, oder der nach dem Verfahren nach einem der Ansprüche 1 bis 12 erhalten werden kann, mit einer biologisch aktiven Substanz in Kontakt gebracht wird.

20. Verfahren zum Herstellen eines biologisch aktivierten Chitosanfilms mit einer erhöhten Zellhaftfähigkeit nach einem der Ansprüche 14 bis 18, das einen Schritt umfaßt, in dem eine biologisch aktive Substanz mit (i) der Chitosanlösung, optional zusammen mit einem biologisch abbaubaren Polymer, in einem Solubilisierungsmedium, das eine wäßrige Lösung von einer Säure umfaßt, in Stufe a) des Verfahrens nach Anspruch 1 in Kontakt gebracht wird, oder alternativ mit (ii) dem Film auf Chitosanbasis mit dem Stabilisierungsmittel in Stufe d) des Verfahrens nach Anspruch 1.

21. Mit einem Film auf Chitosanbasis beschichtetes Produkt, das einen Träger und eine Gesamt- oder Teilbeschichtung des Trägers mit einem Chitosanfilm nach einem der Ansprüche 13 bis 18 umfaßt.

22. Produkt nach Anspruch 21, wobei der Träger aus der Gruppe bestehend aus einer Prothese und medizinisch-chirurgischen Implantaten ausgewählt ist.

23. Produkt nach Anspruch 21, wobei der Träger ein dentales oder traumatologisches Implantat ist und der Film auf Chitosanbasis ein biologisch aktivierter Chitosanfilm ist, der zumindest ein BMP umfaßt.

24. Produkt nach Anspruch 21, wobei der Träger aus der Gruppe bestehend aus Gazen und Matrizen von biokompatiblen und/oder biologisch abbaubaren Polymeren ausgewählt ist, die im Tissue Engineering bzw. in der Zellkulturtechnik verwendet werden.

25. Verwendung eines biologisch aktivierten Chitosanfilms nach einem der Ansprüche 14 bis 18, bei der Entwicklung eines Produkts, das mit dem Film zur Herbeiführung einer biologischen Aktivität in dem aufnehmenden Organismus beschichtet wird, bei der Verbesserung der Osteointegration von Implantaten von dentalem und traumatologischem Nutzen in ihrer Gesamtheit oder in Teilen im Bereich des aufnehmenden Organismus, wo eine Verbesserung und/oder die Regenerierung von Knochengeweben gewünscht wird.

26. Verwendung eines biologisch aktivierten Chitosanfilms nach einem der Ansprüche 14 bis 18, bei der die Entwicklung eines Implantats für einen dentalen oder traumatologischen Verwendungszweck.

## Revendications

1. Procédé de production d'un film à base de chitosan à plus forte capacité d'adhérence cellulaire, qui comprend les étapes qui consistent à :
a) dissoudre du chitosan, facultativement avec un polymère biodégradable, dans un fluide de solubilisation qui comprend une solution aqueuse d'un acide,
b) appliquer sur une surface la solution ainsi obtenue à l'étape a),
c) sécher ladite solution appliquée sur ladite surface pour obtenir un film à base de chitosan,
d) amener ledit film à base de chitosan en contact avec un agent de stabilisation sélectionné parmi (i) une solution aqueuse d'une base, (ii) un tampon de pH égal ou supérieur à 5, (iii) un agent de formation de liaisons et (iv) leurs mélanges,
e) rincer le film stabilisé à base de chitosan obtenu à l'étape d) et
f) sécher ledit film stabilisé à base de chitosan à une température comprise entre 15°C et 80°C en présence d'un écoulement d'air.

2. Procédé selon la revendication 1, dans lequel ledit chitosan présente un poids moléculaire moyen compris entre 150 000 et 2 000 000, de préférence entre 200 000 et 500 000.

3. Procédé selon les revendications 1 ou 2, dans lequel ledit chitosan présente un degré de désacétylation compris entre 65 % et 95 %.

4. Procédé selon la revendication 1, dans lequel ledit chitosan comprend du chitosan dérivé d'un ou plusieurs groupes fonctionnels sélectionnés parmi l'acide phosphonique, un groupe carboxyméthyle, la méthylpyrrolidone et leurs mélanges.

5. Procédé selon la revendication 1, dans lequel ledit fluide de solubilisation comprend une solution aqueuse d'acide organique ou minéral dont le pH est égal ou inférieur à 3,5.

6. Procédé selon la revendication 5, dans lequel ledit acide est sélectionné parmi l'acide chlorhydrique, l'acide acétique, l'acide citrique, l'acide lactique et l'acide malique.

7. Procédé selon la revendication 1, dans lequel le chitosan est dissous avec un polymère biodégradable.

8. Procédé selon la revendication 7, dans lequel ledit polymère biodégradable est sélectionné parmi l'acide polyglycolique, les alginates, les carraghénates, le collagène et leurs mélanges.

9. Procédé selon la revendication 1, dans lequel ledit agent de stabilisation comprend une solution aqueuse d'hydroxyde de sodium, de tampon au phosphate, de tampon au carbonate ou de glutaraldéhyde.

10. Procédé selon la revendication 9, dans lequel ledit agent de stabilisation comprend une solution aqueuse d'hydroxyde de sodium et de glutaraldéhyde.

11. Procédé selon la revendication 9, dans lequel ledit agent de stabilisation comprend (i) un tampon sélectionné parmi un tampon au phosphate et un tampon au carbonate et (ii) du glutaraldéhyde.

12. Procédé selon la revendication 9, dans lequel le séchage du film stabilisé à base de chitosan de l'étape f) est mis en oeuvre à une température comprise entre 20°C et 40°C en présence d'un écoulement d'air.

13. Film à base de chitosan à plus forte capacité d'adhérence cellulaire qui peut être obtenu selon le procédé selon l'une quelconque des revendications 1 à 12, ledit film à base de chitosan à plus forte capacité d'adhérence cellulaire présentant une capacité d'adhérence cellulaire qui est égale ou supérieure à l'augmentation d'au moins 25 % de la valeur de la capacité d'adhérence cellulaire sur un film à base de chitosan non soumis à un traitement d'activation de la capacité d'adhérence cellulaire, ladite capacité d'adhérence cellulaire étant déterminée par l'essai à l'éthidium homodimère ("ethidium homodimer trial" - EHT).

14. Film à base de chitosan activé biologiquement à capacité d'adhérence cellulaire augmentée, qui comprend un film à base de chitosan à plus forte capacité d'adhérence cellulaire selon la revendication 13 et au moins une substance à activité biologique.

15. Film à base de chitosan activé biologiquement selon la revendication 14, dans lequel ladite substance à activité biologique est sélectionnée dans l'ensemble constitué des antibiotiques, des hormones et des protéines à activité biologique dans le corps humain ou animal.

16. Film à base de chitosan activé biologiquement selon la revendication 15, dans lequel ladite substance à activité biologique est la protéine morphogène osseuse ("bone morphogenetic protein" - BMP).

17. Film à base de chitosan activé biologiquement selon la revendication 16, dans lequel ladite BMP est une BMP humaine, naturelle ou recombinée ou ses dimères ou hétérodimères.

18. Film à base de chitosan activé biologiquement selon la revendication 18, dans lequel ladite BMP est sélectionnée parmi la rhBMP-2, la rhBMP-4, la rhBMP-7, leurs dimères ou hétérodimères et leurs mélanges.

19. Procédé de production d'un film à base de chitosan activé biologiquement à capacité d'adhérence cellulaire augmentée selon l'une quelconque des revendications 14 à 18, qui comprend l'étape qui consiste à amener un film à base de chitosan selon la revendication 13 ou qui peut être obtenu selon le procédé selon l'une quelconque des revendications 1 à 12, en contact avec une substance à activité biologique.

20. Procédé de production d'un film à base de chitosan activé biologiquement à capacité d'adhérence cellulaire augmentée selon l'une quelconque des revendications 14 à 18, qui comprend l'étape qui consiste à amener une substance à activité biologique en contact avec (i) ladite solution de chitosan, facultativement avec un polymère biodégradable, dans un fluide de solubilisation qui comprend une solution aqueuse d'un acide de l'étape a) du procédé selon la revendication 1 ou, en variante, avec (ii) le film à base de chitosan en contact avec l'agent de stabilisation de l'étape d) du procédé selon la revendication 1.

21. Produit revêtu d'un film à base de chitosan qui comprend un support et un revêtement total ou partiel dudit support avec le film à base de chitosan selon l'une quelconque des revendications 13 à 18.

22. Produit selon la revendication 21, dans lequel ledit support est sélectionné dans l'ensemble constitué d'implants de prothèse et d'implants médico-chirurgicaux.

23. Produit selon la revendication 21, dans lequel ledit support est un implant dentaire ou traumatologique, ledit film à base de chitosan étant un film à base de chitosan activé biologiquement qui comprend au moins une BMP.

24. Produit selon la revendication 21, dans lequel ledit support est sélectionné dans l'ensemble constitué de gazes et de matrices de polymères biocompatibles et/ou biodégradables utilisées en ingénierie tissulaire.

25. Utilisation d'un film à base de chitosan activé biologiquement selon l'une quelconque des revendications 14 à 18, dans l'élaboration d'un produit revêtu avec ledit film pour entraîner l'activité biologique dans l'organisme récepteur et dans l'amélioration de l'ostéointégration d'implants destinés à une utilisation dentaire ou traumatologique dans leur entièreté ou en partie dans la zone de l'organisme récepteur lorsqu'on souhaite améliorer et/ou régénérer le tissu osseux.

26. Utilisation d'un film à base de chitosan selon l'une quelconque des revendications 14 à 18, dans l'élaboration d'un implant destiné à une utilisation dentaire ou traumatologique.
